# EUROPEAN PATENT SPECIFICATION

(11) **EP 1 852 142 B1**
(45) Date of publication and mention of the grant of the patent: **07.07.2010**
(21) Application number: 06425292.7
(22) Date of filing: 03.05.2006
(51) Int. Cl.: A61M 37/00, A61M 5/20

(54) **Fluid dispensing system**
Flüssigkeitsabgabeeinrichtung
Système de distribution de fluide

(43) Date of publication of application: 07.11.2007
(73) Proprietor: Centre National de la Recherche Scientifique, 75794 Paris Cedex 16 (FR)
(72) Inventor: Hoel, Antonin, 35000 Rennes (FR); Jullien, Marie-Caroline, 35000 Rennes (FR); Mir, Luis Maria, 91370 Verrieres le Buisson (FR)
(74) Representative: Novagraaf Technologies

(56) References cited:
- WO-A-2004/062512
- WO-A2-2005/110525
- US-A1- 2004 092 875

## Description

The present invention relates to a fluid dispensing system.

The micro-techniques allow to realize small sized systems adapted to the deliver of different products. These techniques are particularly useful when products to be delivered are available only on small dose, or when very small quantity of product has to be handled.

The above techniques have a particular advantageous applications in micro-fluidics for the deliver of small quantity of fluids, for instance in medical field. Especially, microfluidics systems can serve in the transdermal administration of drug.

For instance fluid dispensing systems realized according to micro-techniques are known for the delivery of a dose of fluid, in particular a fluid containing large size molecules (DNA for example).

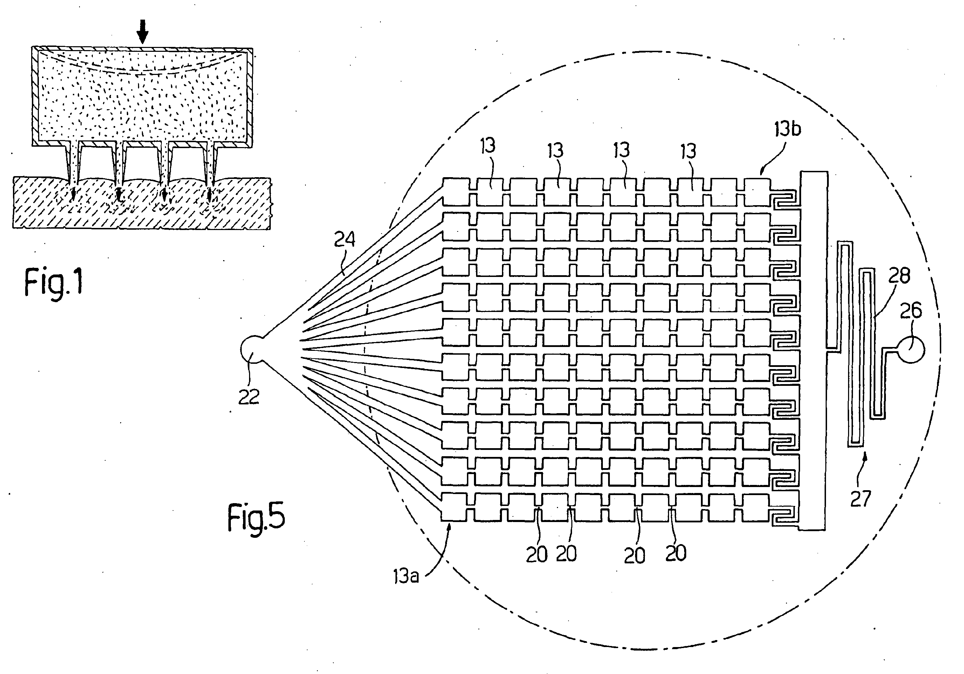

Article of B. Stoeber, D. Liepmann, "Design, Fabrication and Testing of a MEMS Syringe", Technical Digest of the 2002 Solid-State Sensor and Actuator Workshop, Hilton Head Island, South Carolina, U.S.A., June 2-6 2002, pp. 77-80 describes a fluid dispensing system in a form of a multi-needle syringe wherein a deformable, flexible reservoir (figure. 1) on a backside of a array of micro-needles contains a lyophilised drug suspended in non-aqueous fluid. By merely pressing the syringe against the skin it delivers several drops of drug (for instance a vaccine).

The above multi-needles syringe has been designed for wide spread distribution of vaccines in third-word situations where the lack of storage and trained personnel are significant problems.

The scope of the present invention is to realize a fluid dispensing system for supplying to the skin different drops of fluid with great efficacy.

The above scope is realized by the present invention that relates to a fluid dispensing system characterized by comprising a system of interconnected micro chambers communicating with at least one fluid inlet and with at least one suction port; each micro chamber having an output communicating with at least an exit, for example a needle, and having at least one side defined by a deformable membrane subjected to a variable pressure so that a decrease of the pressure with respect to the pressure present at fluid inlet is transferred to the system of micro chambers through the suction port thereby establishing a suction of fluid from the inlet towards the chambers that are filled; an increase of the pressure causing the deflection of the membrane towards the inner side of each micro chamber for the ejection of the fluid contained in each chamber.

By means of a deformable membrane, the common filling reservoir is split into independent delivering chambers, leading to the distribution of all the drops (either of identical volume or of different volumes, depending on chambers geometries) through all the holes, needles or tubes.

The present invention also relates to a fluid dispensing system wherein the system of micro chambers communicates with said at least one fluid inlet at a reference pressure and with a pressure reservoir where pressure may be regulated, with respect to said reference pressure, thereby creating a depression or an increase of pressure, and wherein each chamber having said at least one side defined by said deformable membrane faces the pressure reservoir so that said increase of pressure causes said deflection of the membrane.

The invention shall be described with the help of the attached drawings wherein :
- figure 1 shows a prior art device;
- figure 2 shows a fluid dispensing system in a form of a syringe realized according to the teachings of the present invention ;
- figure 3 shows - in an enlarged scale and in side view - a first detail of two dispensing units of the fluid dispensing system;
- figure 4 shows - in an enlarged scale and in a side view - a second detail of two dispensing units of the fluid dispensing system; and
- figure 5 shows in a top view a variation to the first detail .

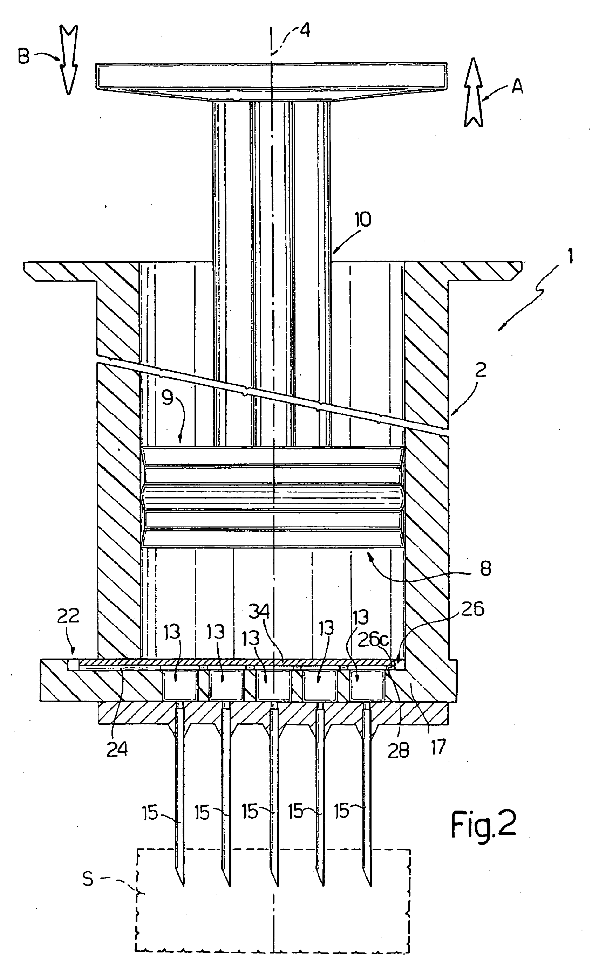

In figure 2 numeral 1 indicates a fluid dispensing system that in the example shown is represented in the form of a multi-needle syringe.

It is however clear that the fluid dispensing system may comprise any pressure reservoir (in the example shown as inner part of a syringe) which allows for generating a low pressure (with respect to a reference pressure external to the filling reservoir, i.e. atmospheric pressure in normal use) and then a high pressure.

With more detail, syringe 1 comprises a main tubular body 2 symmetric along an axis 4 and defining, internally, a cavity 8 coaxial to axis 4. The tubular body 2 may define internally a parallelepiped cavity 8 (preferably) or a cylindrical cavity defining the pressure reservoir.

The syringe 1 comprises a plunger 9 (of known type) slidably moving in the cylindrical cavity 8 along axis 4 and a stem 10 stably connected to plunger 9 and coaxial to axis 4. The main cylindrical body 2, the plunger 9 and the stem 10 are preferably made with not deformable plastic, for instance PVC.

One end of the main cylindrical body 2 carries a system of interconnected micro chambers 13 wherein a liquid may be sucked following a manual action on plunger 9; the system of micro chambers 13 collaborates, in the example shown, with a number of needles15 for the expulsion of the liquid contained in the micro chambers 13. However, each micro chamber may also communicate with a simple hole for the ejection of a fluid or with a tube.

With greater detail, the micro chambers are realized in a microfluidic layer 17 that faces the pressure reservoir 8 by closing an end of the main tubular body 2 and is perpendicular to axis 4.

Preferably microfluidic layer 17 and main cylindrical body 2 are manufactured independently and are further stuck together.

In the embodiment shown (see also figure 5) micro chambers 13 are disposed according to a square array structure wherein each micro chamber 13 communicates, through a communicating channel 20, with at least an adjacent micro chamber 13. More specifically, the micro-chambers disposed along respective lines of the array communicate one with respect to the adjacent other with an interconnecting straight channel 20.

A first number of micro chambers 13a disposed at first ends of the array lines communicates with a fluid inlet 22 through respective conduits 24 extending through microfluidic layer 17 and terminating in a common point defining fluid inlet 22.

Fluid inlet 22 is disposed on the outside of syringe 1 and is in communication with a source of liquid, for instance a drug disposed at atmospheric pressure.

A second number of micro chambers 13b disposed at second ends of the array lines communicates with a suction port 26 through a number of conduits 28 realizing a hydrodynamic resistance to the passage of a liquid.

Suction port 26 communicates with the variable pressure reservoir, in the example cavity 8. More specifically, suction port 26 is a hole through microfluidic layer 17 to make the conduit 28 communicating with the inner of cavity 8.

In order to avoid the liquid to spread non homogeneously into the whole system of micro chambers 13, the addition of the hydrodynamic resistance 28 provides homogeneous filling over all the micro chambers 13.

As it is known, at microscales, liquids tend naturally to flow towards slow hydrodynamic resistive channels. This resistance is roughly proportional to the length of the channel and inversely proportional to the section of the channel. In the case of the chambers array, this means that naturally the liquid tends to flow directly from the inlet to the outlet (shortest pathway), without filling completely all the chambers. By the addition of a hydrodynamic resistance 28 at the exit of the chambers array, the liquid is forced to first explore the lowest resistive pathway (chambers array) before entering the highest resistive pathway (the hydrodynamic resistance 28).

In the embodiment shown the micro chambers 13a are disposed, with respect to the second number of micro chambers 13b, on opposite sides of the array structure having a square shape.

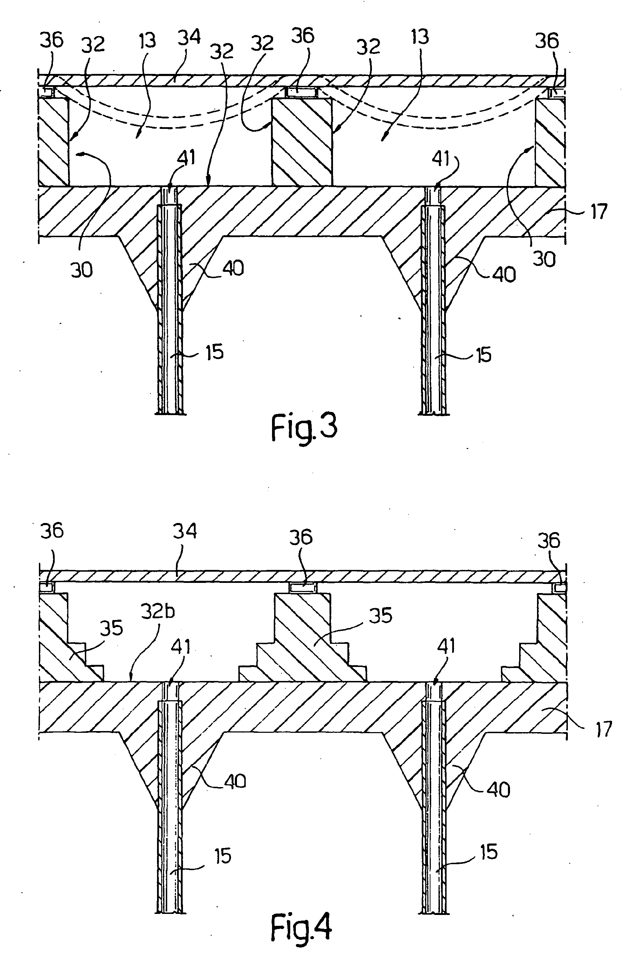

Coming now to figures 3 and 4, in the embodiment shown, each micro chamber 13 defines an internal parallelepiped volume 30 that is limited by surfaces 32 of microfluidic layer 17 and by a deformable elastic membrane 34 that separates the chambers 13 from the cylindrical cavity 8.

Therefore at least one side of each micro chamber 13 is limited by the deformable elastic membrane 34.

The volumes 30 are equal through all the array in the embodiment shown in the figures 3 and 4 but different micro-chambers 13 may also have different volumes.

With more detail, according to the shown embodiment, each micro chamber 13 is defined by a square hole realized in microfluidic layer 17; the deformable membrane 34 resting on square rims 36 of the hole.

Membrane 34 fully covers one side of the micro fluidic layer 17 and the whole system of micro chambers 13, so that the whole membrane is free to deflect towards the system of micro-chambers 13. Rims 36 of each hole may be free or may also be glued with membrane 34.

Membrane 34 is stuck on micro fluidic layer 17 using oxygen plasma at manufacturing stage.

One portion of membrane 34 also faces with a short conduit 26c communicating with the system of micro chambers 13 and forming a part of suction port 26. Under normal pressure conditions, the membrane 34 is substantially flat, the conduit 26c is opened so that the system of micro-chambers 13 is in direct connection to the pressure reservoir 8.

Moreover, as already described with regard to suction port 26, one portion of membrane 34 also faces with a short conduit 22c communicating with the system of micro chambers 13 and forming a part of fluid inlet 22. Under normal pressure conditions, the membrane 34 is substantially flat, the conduit 22c is opened so that the system of micro-chambers 13 is in direct connection to the outside pressure.

Finally microfluidic layer 17 defines on the side towards the exterior of the syringe 1 a number of conical elements 40 each forming a basis for a respective needle 15; the conical elements 40 having a passing hole 41 for the communication of volume 30 with the needle 15.

According to a not shown embodiment, each passing hole 41 may also communicate with an element other than a needle, for instance a tube.

In order to fill the system of micro chambers 13, the stem 10 is pulled according to a first direction (direction A, figure 2) so that a depression is created in the reservoir 8 (in the example cavity 8) of the syringe 1; as above explained, the cavity 8 communicates, through suction port 26, with the system of interconnected micro-chambers 13 so that the pressure in the micro-chambers 13 is also reduced with respect to a reference pressure (atmospheric pressure).

The reduction of pressure in the micro-chambers 13 causes the formation of a pressure gradient between fluid inlet 22 (at reference pressure) and suction port 26 so that a flux of liquid is drawn towards the micro chambers 13 through fluid inlet 22; the fluid first enters in the first micro chambers 13a and then spreads over all micro chambers 13.

During the above operation, the needles 15 are placed in a soft closing material S (shown with dashed line) to close micro chambers 13 to assure the sufficient pressure gradient between inlet 22 and suction port 26. During the above suction operation the elastic membrane may only deflect a limited amount towards the inner of cavity 8 due to the reduction of pressure in the reservoir.

The stem 10 arrives at an end position and the motion of the stem 10 is then reversed according to a second direction (direction B). The movement of the stem 10 is controlled manually: i.e. before the liquid starts to exit from suction port 26, the operator can reverse the motion of the steam 10. In order to inject the liquid, the soft material S has to be removed before reversing the movement of stem 10.

The stem 10 is pushed so that the pressure in the cylindrical cavity 8 is increased; this increase of pressure causes the membrane 34 to deflect (shown with dashed line) and move towards the inner part of each micro chambers 13 so that the fluid contained in each chamber 13 is ejected through the needle 15.

Moreover the membrane 34 deflects so that it sets down the peripheral rims 36 of any micro chamber that is closed during the ejection of the fluid.

This process of closing of all the chambers, independently one from each other, prevents from leaks from adjacent chambers 13; this process further prevent any leak through inlet and outlet while injecting without the need of closing of fluid inlet 22 and suction port 26.

Accordingly, each needle 15 outputs an amount of fluid; if the micro-chambers 13 have the same volume each needles (or tubes or holes) 15 outputs the same quantity of fluid (homogeneous output) with the same pressure, conversely if the chambers have different volumes different needles (or tubes or holes) 15 output different quantity of fluid (heterogeneous output). In all the cases the invention provides the control of the volume dispensed by each individual chamber.

The fluid dispensing system 1 ensures the ejection of the content of each micro chamber 13 (whether all chambers 13 have the same volume or not) through the exits (needles 15, or holes or tubes), whatever the pressure outside the exits as long as the outside pressure is lower than the reservoir pressure and at the same order of magnitude from one exit to the other.

According to the embodiment of figure 4 the bottom surface 32b of each micro-chamber 13 has a pyramidal structure 35 in order to lower the remaining liquid into the device after injection while deflecting membrane 34. In other words, the section of the chamber 13 decreases from the side defined by elastic membrane 34 towards the output 41 communicating with the needle 15.

The advantages of the invention are that it realizes a fluid dispensing system for supplying different drops of fluid with great efficacy and control. In fact, the distribution of all the drops (either of identical volume or of different volumes) through needles 15 is ensured by means of a single deformable membrane 34 acting on a number of independent chambers 13 by closing the chambers 13 and obtaining the ejection of the fluid, wherein the ejection from each chamber is independent from the ejection obtained in other chambers.

Moreover, the invention provides the control of the volume dispensed by each of the individual chambers.

## Claims

1. Fluid dispensing system **characterized by** comprising a system of interconnected micro chambers (13) communicating with at least one fluid inlet (22) and with at least one suction port (26);
each micro chamber (13) having an output communicating with at least an exit, for example a needle, (15) and having at least one side defined by a deformable membrane (34) subjected to a variable pressure so that a decrease of the pressure with respect to the pressure present at fluid inlet (22) is transferred to the system of micro chambers (13) through the suction port (26) thereby establishing a suction of fluid from the inlet (22) towards the chambers (13) that are filled;
an increase of the pressure causing the deflection of the membrane (34) towards the inner side of each micro chamber for the ejection of the fluid contained in each chamber (13).

2. Fluid dispensing system as claimed in claim 1, wherein each chamber is closed individually by said membrane upon increase of the pressure.

3. Fluid dispensing system as claimed in claim 1, wherein said system of micro chambers comprises chambers having each the same size.

4. Fluid dispensing system as claimed in claim 1, wherein said system of micro chambers comprises chambers having different sizes.

5. Fluid dispensing system as claimed in claim 1, wherein said system of micro chambers comprises chambers disposed according to a network structure.

6. Fluid dispensing system as claimed in claim 1, wherein said micro-chambers are disposed along respective lines of an array and communicate one with respect to the adjacent other with interconnecting channels (20).

7. Fluid dispensing system as claimed in claim 1, wherein the section of each micro-chamber decreases from the side defined by elastic membrane (34) towards the output communicating with said exit (15).

8. Fluid dispensing system as claimed in claim 1, wherein a hydraulic resistance (28) is disposed between at least one micro chamber (13b) and said suction port (26); said hydraulic resistance (28) provides homogeneous filling over all the micro chambers (13).

9. Fluid dispensing system as claimed in claim 1, wherein the system of micro chambers (13) communicates with said at least one fluid inlet (22) at a reference pressure and with a pressure reservoir (8) where pressure may be regulated, with respect to said reference pressure, thereby creating a depression or an increase of pressure, and wherein each chamber (13) having said at least one side defined by said deformable membrane (34) faces the pressure reservoir (8) so that said increase of pressure causes said deflection of the membrane (34).

10. Fluid dispensing system as claimed in claim 9, wherein the system of micro chambers (13) comprises a plurality of micro chambers disposed according a network structure.

11. Fluid dispensing system as claimed in claim 9, wherein the system of micro chambers (13) comprises a first plurality of micro chambers (13a) communicating (24) with said fluid inlet (22) and a second plurality of micro chambers (13b) communicating (28) with said suction port (26).

12. Fluid dispensing system as claimed in as claimed in claim 11, wherein said first plurality of micro chambers (13a) and said second plurality of micro chambers (13b) are disposed on opposite side of an array structure.

13. Fluid dispensing system as claimed in claim 9, wherein the system of micro chambers (13) comprises a first plurality of micro chambers (13a) communicating (24) with said fluid inlet (22) and a second plurality of micro chambers (13b) communicating (28) with said suction port (26) through an hydrodynamic resistance (27).

## Patentansprüche

1. Flüssigkeitsspendersystem, **dadurch gekennzeichnet, daß** es ein System miteinander verbundener Mikrokammern (13) umfaßt, die mit mindestens einem Flüssigkeitseinlaß (22) und mit mindestens einem Saugauslaß (26) in Verbindung stehen,
wobei jede Mikrokammer (13) einen Auslaß hat, der mit mindestens einem Ausgang, zum Beispiel einer Nadel (15), in Verbindung steht, und von der mindestens eine Seite aus einer verformbaren Membran (34) definiert wird, die variablem Druck unterliegt, so daß eine Abnahme des Drucks hinsichtlich des am Flüssigkeitseinlaß (22) anliegenden Drucks über den Saugauslaß (26) an das Mikrokammersystem (13) übertragen wird und somit ein Absaugen von Flüssigkeit vom Einlaß (22) zu den Kammern (13) entsteht, die gefüllt werden,
wobei ein Anstieg des Drucks das Durchbiegen der Membran (34) zur Innenseite jeder Mikrokammer für den Ausstoß der in jeder Kammer (13) enthaltenen Flüssigkeit verursacht.

2. Flüssigkeitsspendersystem nach Anspruch 1, **dadurch gekennzeichnet, daß** jede Kammer durch die besagte Membran bei Anstieg des Drucks individuell geschlossen wird.

3. Flüssigkeitsspendersystem nach Anspruch 1, **dadurch gekennzeichnet, daß** das besagte Mikrokammersystem Kammern umfaßt, die jeweils die gleiche Größe haben.

4. Flüssigkeitsspendersystem nach Anspruch 1, **dadurch gekennzeichnet, daß** das besagte Mikrokammersystem Kammern umfaßt, die unterschiedliche Größen haben.

5. Flüssigkeitsspendersystem nach Anspruch 1, **dadurch gekennzeichnet, daß** das besagte Mikrokammersystem Kammern umfaßt, die in einer Netzstruktur angeordnet sind.

6. Flüssigkeitsspendersystem nach Anspruch 1, **dadurch gekennzeichnet, daß** die besagten Mikrokammern entlang den jeweiligen Linien einer Matrix angeordnet sind und mit der jeweils anschließenden Kammer über Verbindungskanäle (20) in Verbindung stehen.

7. Flüssigkeitsspendersystem nach Anspruch 1, **dadurch gekennzeichnet, daß** der Querschnitt jeder Mikrokammer von der durch die elastische Membran (34) definierten Seite her zum Ausgang hin abnimmt, der mit dem besagten Auslaß (15) in Verbindung steht.

8. Flüssigkeitsspendersystem nach Anspruch 1, **dadurch gekennzeichnet, daß** ein hydraulischer Widerstand (28) zwischen mindestens einer Mikrokammer (13b) und dem besagten Saugauslaß (26) angeordnet ist, wobei der besagte hydraulische Widerstand (28) für homogene Befüllung in allen Mikrokammern (13) sorgt.

9. Flüssigkeitsspendersystem nach Anspruch 1, **dadurch gekennzeichnet, daß** das Mikrokammersystem (13) mit besagtem mindestens einen Flüssigkeitseinlaß (22) bei einem Bezugsdruck in Verbindung steht und mit einem Drucktank (8), wo der Druck nach dem besagten Bezugsdruck geregelt werden kann, und **dadurch** einen Unterdruck oder einen Druckanstieg schafft, und **dadurch**, daß jede Kammer (13), deren besagte mindestens eine Seite durch die besagte verformbare Membran (34) definiert ist, dem Drucktank (8) gegenüber liegt, so daß der besagte Druckanstieg die besagte Durchbiegung der Membran (34) verursacht.

10. Flüssigkeitsspendersystem nach Anspruch 9, **dadurch gekennzeichnet, daß** das Mikrokammersystem (13) mehrere Mikrokammern umfaßt, die in einer Netzstruktur angeordnet sind.

11. Flüssigkeitsspendersystem nach Anspruch 9, **dadurch gekennzeichnet, daß** das Mikrokammersystem (13) eine erste Mehrzahl von Mikrokammern (13a) umfaßt, die mit dem besagten Flüssigkeitseinlaß (22) in Verbindung (24) stehen, und eine zweite Mehrzahl von Mikrokammern (13b), die mit dem besagten Saugauslaß (26) in Verbindung (24) stehen.

12. Flüssigkeitsspendersystem nach Anspruch 11, **dadurch gekennzeichnet, daß** die erste Mehrzahl von Mikrokammern (13a) und die besagte zweite Mehrzahl von Mikrokammern (13b) auf der entgegengesetzten Seite einer Matrixstruktur angeordnet sind.

13. Flüssigkeitsspendersystem nach Anspruch 9, **dadurch gekennzeichnet, daß** das Mikrokammersystem (13) eine erste Mehrzahl von Mikrokammern (13a) umfaßt, die mit dem besagten Flüssigkeitseinlaß (22) in Verbindung (24) stehen, und eine zweite Mehrzahl von Mikrokammern (13b), die mit dem besagten Saugauslaß (26) durch einen hydrodynamischen Widerstand (27) in Verbindung (28) stehen.

## Revendications

1. Système de distribution de fluide **caractérisé en ce qu'**il comprend un système de micro-chambres reliées entre elles (13) communiquant avec au moins une entrée de fluide (22) et munies d'au moins un port d'aspiration (26) ;
chaque micro-chambre (13) ayant une sortie communiquant avec au moins un dégagement, par exemple une aiguille (15) et ayant au moins un côté défini par une membrane déformable (34) soumise à une pression variable de telle sorte qu'une diminution de la pression par rapport à la pression présente au niveau de l'entrée de fluide (22) est transférée au système de micro-chambres (13) par l'intermédiaire du port d'aspiration (26) en établissant ainsi une aspiration du fluide entre l'entrée (22) et les chambres (13) qui sont remplies ;
une augmentation de la pression provoquant un fléchissement de la membrane (34) vers l'intérieur de chaque micro-chambre pour l'éjection du fluide contenu dans chaque chambre (13).

2. Système de distribution de fluide tel que revendiqué dans la revendication 1, dans lequel chaque chambre est fermée individuellement par ladite membrane au moment de l'augmentation de la pression.

3. Système de distribution de fluide tel que revendiqué dans la revendication 1, dans lequel ledit système de micro-chambres comprend des chambres ayant chacune la même taille.

4. Système de distribution de fluide tel que revendiqué dans la revendication 1, dans lequel ledit système de micro-chambres comprend des chambres ayant des tailles différentes.

5. Système de distribution de fluide tel que revendiqué dans la revendication 1, dans lequel ledit système de micro-chambres comprend des chambres disposées selon une structure de réseau.

6. Système de distribution de fluide tel que revendiqué dans la revendication 1, dans lequel lesdites micro-chambres sont disposées le long de lignes respectives d'un groupe et communiquent l'une avec l'autre adjacente par des canaux d'interconnexion (20).

7. Système de distribution de fluide tel que revendiqué dans la revendication 1, dans lequel la section de chaque micro-chambre diminue depuis le côté défini par la membrane élastique (34) vers la sortie communiquant avec ledit dégagement (15).

8. Système de distribution de fluide tel que revendiqué dans la revendication 1, dans lequel une résistance hydraulique (28) est disposée entre au moins une micro-chambre (13b) et ledit port d'aspiration (26) ; ladite résistance hydraulique (28) fournissant un remplissage homogène sur l'ensemble des micro-chambres (13).

9. Système de distribution de fluide tel que revendiqué dans la revendication 1, dans lequel le système de micro-chambres (13) communique avec ladite au moins une entrée de fluide (22) sous une pression de référence et avec un réservoir de pression (8) dans lequel la pression peut être régulée par rapport à ladite pression de référence, créant ainsi une dépression ou une augmentation de la pression et dans lequel chaque chambre (13) ayant ledit au moins un côté défini par ladite membrane déformable (34) fait face au réservoir de pression (8) de telle sorte que ladite augmentation de pression provoque ledit fléchissement de la membrane (34).

10. Système de distribution de fluide tel que revendiqué dans la revendication 9, dans lequel le système de micro-chambres (13) comprend une pluralité de micro-chambres disposées selon une structure de réseau.

11. Système de distribution de fluide tel que revendiqué dans la revendication 9, dans lequel le système de micro-chambres (13) comprend une première pluralité de micro-chambres (13a) communiquant (24) avec ladite entrée de fluide (22) et une seconde pluralité de micro-chambres (13b) communiquant (28) avec ledit port d'aspiration (26).

12. Système de distribution de fluide tel que revendiqué dans la revendication 11, dans lequel ladite première pluralité de micro-chambres (13a) et ladite seconde pluralité de micro-chambres (13b) sont disposées sur le côté opposé d'une structure de groupe.

13. Système de distribution de fluide tel que revendiqué dans la revendication 9, dans lequel le système de micro-chambres (13) comprend une première pluralité de micro-chambres (13a) communiquant (24) avec ladite entrée de fluide (22) et une seconde pluralité de micro-chambres (13b) communiquant (28) avec ledit port d'aspiration (26) par l'intermédiaire d'une résistance hydrodynamique (27).
